# EUROPEAN PATENT APPLICATION

(11) **EP 3 366 211 A1**
(43) Date of publication of application: **29.08.2018**
(21) Application number: 17158134.1
(22) Date of filing: 27.02.2017
(51) Int. Cl.: A61B 5/08, A61B 5/113, A61B 5/00, G01B 7/16

(54) **WEARABLE DEVICE**

(71) Applicant: Koninklijke Philips N.V., 5656 AE Eindhoven (NL)
(72) Inventor: BONGERS, Edwin Gerardus Johannus Maria, 5656 AE Eindhoven (NL)
(74) Representative: de Haan, Poul Erik

(57) **Abstract**

The described embodiments relate to wearable devices (104, 200) that can collect data related to the stretching and compressing of a meander or flexible inductor (108, 116, 216, 306) of the wearable device. By collecting such data, the wearable device can track the motions of a person (102) that is wearing the wearable device. Furthermore, the wearable device can determine whether it is becoming loose from the body of the person, or whether components of the wearable device are degrading. Flexible inductor(s) (108, 116, 216, 306) in the wearable device can be used to measure changes in inductance and/or impedance that can occur when the flexible inductor(s) are stretched or compressed. Signals resulting from these changes can be filtered to identify types of movements occurring at the wearable device.

## Description

### Technical Field

The described embodiments relate to generally to wearable computing devices. More particularly, but not exclusively, the various methods and apparatuses disclosed herein relate to wearable devices that include one or more flexible inductors (sometimes referred to as "meanders") for determining certain metrics related to the wearable device and the person wearing the wearable device.

### Background

Wearable devices can be used to collect data about a person wearing a wearable device. Typically, the wearable device can rely on being proximate to a person's body in order to gauge certain metrics about the health and activities of the person. Unfortunately, because of how often people move on a daily basis, many wearable devices-particularly those that are adhered to skin using biocompatible adhesives-become loose and do not stay in their intended location. As a result, many wearable devices can provide inaccurate data because they cannot adequately collect data about a person outside of their intended position. Furthermore, it may not be apparent to a user that the wearable device has moved from its intended position, therefore, the person may not know whether to correct a position of the wearable device.

### Summary

The present disclosure is directed wearable devices. Specifically, the described embodiments relate to wearable devices that can have one or more flexible inductors for gauging certain features about the wearable device and collecting data about the person wearing the wearable device.

In some embodiments, a wearable apparatus is set forth as including: a flexible inductor configured to modify an initial signal (e.g., a sine wave) to produce a modified signal when the flexible inductor is stretched while receiving the initial signal; a controller configured to provide the initial signal to the flexible inductor and determine whether the flexible inductor has been stretched by at least demodulating the modified signal; and a flexible body configured to at least partially envelope the flexible inductor and the controller.

In various embodiments, the wearable apparatus may further include a memory device that is connected to the controller. The memory device may be configured to receive respiratory rate data from the controller when a length of the flexible inductor oscillates over time. In various embodiments, the wearable apparatus may further include a conductive contact located at an exterior surface of the flexible body. The conductive contact may be configured to receive a monitoring signal from the controller. The controller may be further configured to determine when the conductive contact has shifted from an original position on a person by at least monitoring a change of impedance of the flexible inductor.

In various embodiments, the controller may be further configured to determine whether a material of the flexible inductor is degrading by taking impedance measurements of the flexible inductor at different times. In various versions, the wearable apparatus may further include a wireless transmitter configured to output a warning signal to a computing device when the impedance measurements exceed a threshold impedance value for a period of time.

In various embodiments, the wearable apparatus may further include a wireless transmitter configured to output a wireless signal to a computing device when the controller determines that the flexible inductor has been stretched past a predetermined length. The wireless signal can indicate an arrangement of the flexible inductor to the computing device. In some embodiments, the controller can be configured to determine, at least partially based on the demodulating of the modified signal, a length by which the wearable apparatus is being stretched. Furthermore, the wearable apparatus can include a sensor, and the controller can modify a data collection operation of the sensor when the length satisfies a predetermined threshold. Determining the length can include determining a change of inductance of the flexible inductor when the flexible inductor is stretched. In various embodiments, the wearable device can include a memory. The controller can modify a rate value stored in the memory when the length exceeds the predetermined threshold. The rate value can indicate an average number of times the wearable apparatus is stretched within a period of time.

In various embodiments, the controller can include at least two electrodes connected to the controller and located at opposite ends of the flexible body. The at least two electrodes can become less proximate when the flexible inductor is stretched. The controller can simultaneously monitor a vital sign of a person using the electrodes and track the stretching of the flexible inductor. Furthermore, the controller can optionally determine a frequency of the change of inductance of the flexible inductor and/or determine whether the at least two electrodes have separated beyond a threshold distance based on the frequency of the change of inductance. In some embodiments, the controller can change an operation of at least two electrodes when the at least two electrodes have separated beyond the threshold distance. In yet other embodiments, the wearable device can include a flexible inductor that carries the modified signal when the flexible inductor is receiving the initial signal.

In another aspect, a method of using a wearable device is set forth. The wearable device can include a flexible body that at least partially envelopes a flexible inductor and a controller. The method can include steps of: transmitting, by the controller, an initial signal to the flexible inductor; modifying, by the flexible inductor, the initial signal to produce a modified signal when the flexible inductor is stretched while receiving the initial signal; and determining, by the controller, whether the flexible inductor has been stretched by at least demodulating the modified signal.

In yet another aspect, a system may include: a flexible body that houses at least two electrodes and a flexible inductor, the flexible inductor configured to transmit a sinusoidal signal when the flexible inductor is in a stretched state; a controller configured to receive a resulting signal based on the flexible inductor receiving the sinusoidal signal and filter the resulting signal; and a transmitter configured to transmit a wireless signal when the resulting signal indicates that the at least two electrodes have separated beyond a threshold distance.

In various embodiments, the controller of the system may be further configured to determine a change of inductance of the flexible inductor. In various embodiments, the controller may be further configured to determine a frequency of the change of inductance of the flexible inductor and determine whether the at least two electrodes have separated beyond the threshold distance based on the frequency of the change of inductance.

In various embodiments, the controller may be further configured to change an operation of the at least two electrodes when the at least two electrodes have separated beyond the threshold distance. In various embodiments, the system may further include another flexible inductor that carries the resulting signal when the flexible inductor is receiving the sinusoidal signal.

The term "controller" is used herein generally to describe various apparatus relating to the operation of one or more components described herein. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media (generically referred to herein as "memory," e.g., volatile and non-volatile computer memory such as RAM, PROM, EPROM, and EEPROM, floppy disks, compact disks, optical disks, magnetic tape, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present invention discussed herein. The terms "program" or "computer program" are used herein in a generic sense to refer to any type of computer code (e.g., software or microcode) that can be employed to program one or more processors or controllers.

In one network implementation, one or more devices coupled to a network may serve as a controller for one or more other devices coupled to the network (e.g., in a master/slave relationship). In another implementation, a networked environment may include one or more dedicated controllers that are configured to control one or more of the devices coupled to the network. Generally, multiple devices coupled to the network each may have access to data that is present on the communications medium or media; however, a given device may be "addressable" in that it is configured to selectively exchange data with (i.e., receive data from and/or transmit data to) the network, based, for example, on one or more particular identifiers (e.g., "addresses") assigned to it.

The term "network" as used herein refers to any interconnection of two or more devices (including controllers or processors) that facilitates the transport of information (e.g., for device control, data storage, data exchange, etc.) between any two or more devices and/or among multiple devices coupled to the network. As should be readily appreciated, various implementations of networks suitable for interconnecting multiple devices may include any of a variety of network topologies and employ any of a variety of communication protocols. Additionally, in various networks according to the present disclosure, any one connection between two devices may represent a dedicated connection between the two systems, or alternatively a non-dedicated connection. In addition to carrying information intended for the two devices, such a non-dedicated connection may carry information not necessarily intended for either of the two devices (e.g., an open network connection). Furthermore, it should be readily appreciated that various networks of devices as discussed herein may employ one or more wireless, wire/cable, and/or fiber optic links to facilitate information transport throughout the network.

The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, a mouse, keyboard, keypad, various types of game controllers (e.g., joysticks), track balls, display screens, various types of graphical user interfaces (GUIs), touch screens, microphones and other types of sensors that may receive some form of human-generated stimulus and generate a signal in response thereto.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

### Brief Description of the Drawings

In the drawings, like reference characters generally refer to the same parts throughout the different views. Also, the drawings are not necessarily to scale, emphasis instead generally being placed upon illustrating the principles of the invention.
FIGS. 1A-1C illustrate a wearable device that can be worn by a person, and components of the wearable device.
FIGS. 2A and 2B illustrate embodiments of a wearable device that can include electrode contacts and a controller that tracks changes to one or more flexible inductors of the wearable device.
FIG. 3 illustrates a system diagram of a controller that can be included in any of the wearable devices discussed herein.
FIGS. 4A and 4B illustrate plots of changes in inductance that can occur at one or more of the flexible inductors discussed herein.
FIGS. 5A and 5B illustrate plots that can represent changes in inductance that can occur when one or more flexible inductors of a wearable device are in a stretched state.
FIG. 6 illustrates a method for determining how a person is moving based on changes of inductance that can occur at a flexible inductor of a wearable device.
FIG. 7 illustrates a method for identifying a configuration of a wearable device using one or more flexible inductors of the wearable device.
FIG. 8 illustrates a method for determining whether one or more flexible inductors of a wearable device are experiencing degradation.
FIG. 9 illustrates a method for determining whether one or more flexible inductors of a wearable device have been stretched.

### Detailed Description

The described embodiments relate to wearable computing devices that can be worn by a person to monitor daily activities and perform health diagnostics. Many wearable devices rely on biocompatible adhesives to maintain their position on a person's body. Unfortunately, many wearable devices can become loose or shift from their intended position, thereby affecting the performance of the wearable device. For example, a wearable device that measures a condition of the body may require that a sensor be proximate to an organ on the body. Therefore, when the wearable device shifts from being proximate to the organ, the wearable device may provide data that is inaccurate or otherwise fail to perform its intended function.

The described embodiments relate to a wearable device that can detect when the wearable device has become loose or shifted from its original or intended location. The wearable device can detect whether the wearable device has shifted or become loose by using a conductive meander and/or flexible inductor. The flexible inductor can carry a signal that experiences changes as a result of the flexible inductor moving, stretching, and/or compressing. The signal can be generated (e.g., modulated) at a controller (e.g., one or more processors) of the wearable device and the changes to the signal can be based on changes in impedance and/or inductance that occur when the flexible inductor moves, stretches, and/or condenses. Nominal and substantial changes in impedance and/or inductance can each be indicative of different types of movements occurring at the wearable device. For example, a nominal stretch can be due to a subtle movement of the body whereas a substantial stretch can be due to respiratory activity when the wearable device is wrapped at least partially around the chest cavity of a person. The signal can be a modulated sinusoidal signal that is applied across the flexible inductor and demodulated in order to identify changes in impedance and/or inductance of the flexible inductor.

In various embodiments, demodulation can be performed using quadrature demodulation, which can identify an in-phase component and a quadrature component of the signal resulting from movements of the flexible inductor. The in-phase component can correspond to a real variable of the resulting signal and the quadrature component can correspond to an imaginary variable of the resulting signal. Each component of the signal can be used to differentiate between types of movements occurring at the wearable device. A controller of the wearable device can assist in differentiating between the types of movements. The controller can include a signal output that provides one or more sinusoidal signals to the flexible inductor, and/or a signal input that can receive the resulting sinusoidal signal that is output by the flexible inductor. In some embodiments, the wearable device can include multiple flexible inductors that are connected to the controller. For example, a signal can be applied to a first flexible inductor, and a resulting signal can be induced in a second flexible inductor. The resulting signal can be measured at the second flexible inductor and thereafter demodulated to identify types of movements occurring at the first and/or second flexible inductor.

The controller can further include an analog-to-digital converter that converts the resulting signal into a digital signal. Thereafter, the resulting signal can be demodulated by the controller to remove the initial signal that was applied to the flexible inductor. By removing the initial signal through demodulation, signal artifacts can be identified and associated with one or more types of movements of the wearable device. In some embodiments, at least one filter can be applied to the in-phase component of the resulting signal and at least one other filter can be applied to the quadrature component of the resulting signal. Changes in the resulting signal can be tracked over time to identify other properties of the wearable device. For example, changes in the resulting signal over time can be indicative of degradation of the flexible inductor, components, and/or material from which the wearable device is made.

The wearable device can include one or more of sensors, actuators, image capture devices, speed recognition devices, position recognition devices, networking devices, display devices, flexible electronics, optical electronics, and/or any other apparatuses suitable for operating a portable electronic device. The wearable device can be used for fitness and health monitoring, entertainment, enterprise and/or industrial management. The wearable device can monitor vital signs of a body such as respiratory rate, heart rate, skin temperature, and/or perform electrocardiography. In order for the wearable to device to detect certain vital signs, the wearable device can include one or more contacts, such as gel contacts, that can send and receive signals from one or more controllers of the wearable device. The wearable device can be made from a flexible material that allows the wearable device to be held in place despite a person moving while wearing the wearable device.

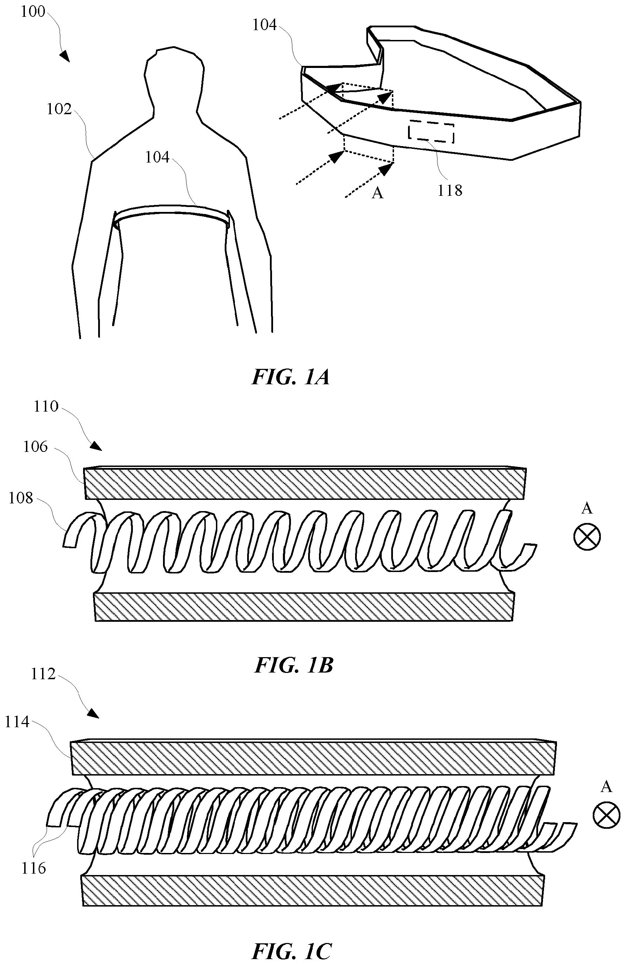

FIG. 1A illustrates a diagram 100 that provides a wearable device 104 that is worn by a person 102. The wearable device 104 can be worn by the person 102 at different locations on the body of the person 102. For example, as illustrated in diagram 100, the wearable device 104 can be worn around the chest of the person 102 in order to track respiratory data or other data related to the person 102. The wearable device 104 can be a single loop or a dual-end strap that can be connected to form a loop. Furthermore, the wearable device 104 can include one or more controllers 118 (e.g., with one or more processors, ASIC, FPGA, etc.) that are connected to one or more flexible inductors. The flexible inductor can extend at least partially over a portion of the body of the person 102 and change configuration when the body moves. When the flexible inductor is receiving a signal and the configuration of the flexible inductor changes, a resulting signal can be measured and compared to the original signal to identify how the configuration of the flexible inductor has changed. For example, when the wearable device 104 is stretched across the body of the person 102, the changes in the configuration of the flexible inductor can correspond to respiratory movements of the person. When the respiratory movements occur multiple times over a period observed by the controller 118 of the wearable device 104, a respiratory rate can be recorded and/or transmitted by the controller 118.

FIG. 1A further includes a cross-section A that illustrates the flexible inductor inside a body of the wearable device 104. FIG. 1B illustrates a diagram 110 that provides details of the cross-section A of in embodiment of the wearable device 104. The diagram 110 can include a layer 106 of the wearable device 104 that can at least partially envelope a flexible inductor 108 that can extend through the wearable device 104. The flexible inductor 108 can be made from one or more wires that may or may not be coated with a non-conductive material. In some embodiments, the flexible inductor 108 is adhered and/or sewn to the layer 106 such that the flexible inductor 108 can extend and compress with the layer 106 when the wearable device 104 is stretched or compressed. In some embodiments, the layer 106 can include grounded conductive material that creates a faraday cage to prevent external electromagnetic fields from influencing a signal that is carried by the flexible inductor 108. The grounded conductive layer of the layer 106 can be controlled by the controller 118 of the wearable device 104 and act to turn off and on the faraday cage depending on whether the wearable device 104 is being worn or not. In some embodiments, the wearable device 104 can include multiple flexible inductors 116, as provided in diagram 112 of FIG. 1C. The multiple flexible inductors 116 can be wrapped about an axis that is collinear with respect to each flexible inductor of the multiple flexible inductors 116. Alternatively, in some embodiments, each flexible inductor of the multiple flexible inductors 116 can be at least partially interweaved but wrapped about different axes that are parallel. Furthermore, in other embodiments, each flexible inductor of the multiple flexible inductors 116 can be separated such that their coils do not overlap. In each of the embodiments of the multiple flexible inductors 116, a signal that is transmitted into one of the flexible inductors can induce a resulting signal in another flexible inductor. The resulting signal can be indicative of changes that are occurring in the configuration of the wearable device 104 and/or how much the wearable device 104 is being stretched and/or condensed.

In some embodiments, the wearable device 104 can include multiple sections of multiple flexible inductors 116 that are separately monitored by one or more controllers 118 of the wearable device 104. Each of the flexible inductors can extend at least partially through a circumference of the wearable device 104. In this way, changes to configurations of individual flexible inductors can be monitored to identify how the wearable device 104 is being used and/or provide feedback according to the changes in the configurations. For example, the wearable device 104 can be wrapped around the chest of the person 102 and the controller 118 of the wearable device 104 can monitor a respiratory rate of the person 102. Simultaneously, the controller 118 can monitor a section of flexible inductor that is most proximate to the heart of the person 102 compared to other flexible inductors of the wearable device 104. By separately analyzing a resulting signal at the section of flexible inductor near the heart, the controller 118 can determine a heart rate of the person 102. It should be noted that the vector symbol and "A" in FIG. 1B and FIG. 1C correspond to the direction arrows "A" in FIG. 1A, and therefore indicate where the cross sections of FIG. 1B and FIG. 1C are located in the wearable device 104 of FIG. 1A.

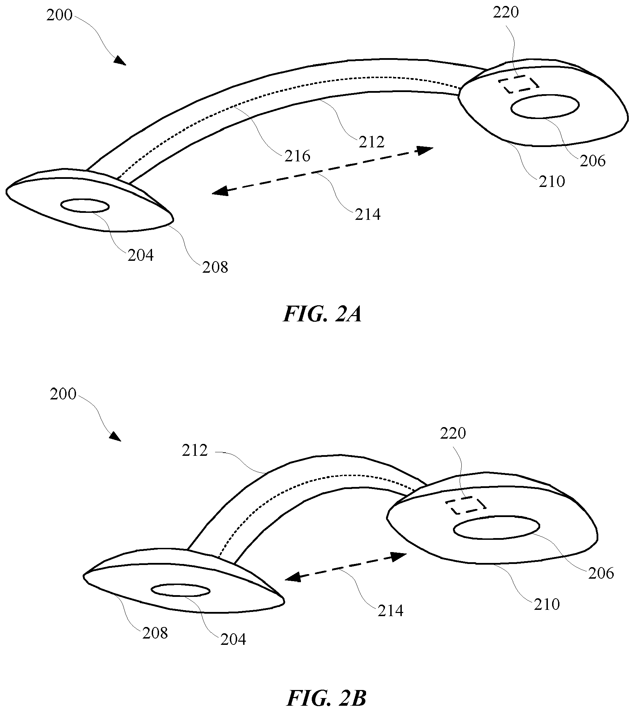

FIGS. 2A and 2B illustrate embodiments of a wearable device 200 that can include electrode contacts and a controller 220 that tracks movement of one or more flexible inductors 216 that extend between the electrode contacts. The wearable device 200 can include a first electrode 204 (also referred to as a conductive contact) at a first end 208 and a second electrode 206 at a second end 210 of the wearable device 200. Each of the first electrode 204 and the second electrode 206 can be connected to the controller 220, which can be located in the first end 208 and/or the second end 210. The first electrode 204 and the second electrode 206 can be used to transmit a signal into the body of a person 102 and/or measure characteristics of the body of the person 102. For example, the first electrode 204 and the second electrode 206 can each be part of a sensor that detects features of the body of the person 102 and transmits signals to the controller 220 of the wearable device 200. A portion of the first end 208 and the second end 210 can include an adhesive surface that can adhere to the surface of a body of the person 102 in order that the first electrode 204 and the second electrode 206 will remain in place during a monitoring process. Unfortunately, given the mobility and frequent motions of the person 102, the wearable device 200 can become loose and/or shift on the body of the person 102, e.g., due to degradation of a biocompatible adhesive used to adhere the wearable device 200 to the person's body. Additionally, the wearable device 200 can be intended to flex and shift while the person 102 is wearing the wearable device 200 in order to perform fitness tracking. The controller 220 of the wearable device 200 can detect such shifting using one or more flexible inductors 216 disposed within a flexible body 212 of the wearable device 200.

As provided in FIG. 2B, a distance 214 between the first end 208 and the second end 210 can become larger and/or smaller according to motions of the person 102 wearing the wearable device 200. In order to detect changes in the distance 214, the wearable device 200 can include one or more flexible inductors 216 that can carry a signal transmitted by the controller 220 of the wearable device 200. Because the flexible inductor 216 can change its configuration as a result of the changes in the distance 214, impedance and/or inductance of the flexible inductor 216 can also change. These changes in the impedance and/or inductance can be measured by the controller 220 of the wearable device 200 and used to determine how the wearable device 200 is changing position. For example, as shown in FIG. 2A, when the wearable device 200 is in a stretched state, the flexible inductor 216 extends in a more linear manner thereby modifying the inductance of the flexible inductor 216 accordingly. Furthermore, as shown in FIG. 2B, when the wearable device 200 is in a compressed state, the flexible inductor 216 is in a less linear manner thereby modifying the inductance of the flexible inductor 216 accordingly. Each of these configurations of the flexible inductor 216 uniquely affect any signal that is transmitted over the flexible inductor 216. Moreover, when there are multiple flexible inductors 216 in the body 212 of the wearable device 200, a signal transmitted in flexible inductor can induce a resulting signal in another flexible inductor. The resulting signal can thereafter be analyzed by the controller 220 to identify features that are indicative of a position or movement of the wearable device 200.

In some embodiments, the body 212 can include a mesh conductive material that is grounded to create a faraday cage around the flexible inductor 216. The mesh conductive material can be flexible and stretchable to allow the wearable device 200 to be stretched and attached to the body of a person 102. The controller 220 can be disposed within the first end 208 and/or the second end 210, and be tasked with providing a signal to at least one flexible inductor in the body 212 and measure a resulting signal in another flexible inductor in the body 212. In some embodiments, a first controller in the first end 208 can transmit a wireless signal to a second controller in the second end 210. The wireless signal can be used to gauge the distance 214 between the first end 208 and the second end 210. Furthermore, the wireless signal can be used to determine whether an orientation of the first end 208 relative to the second end 210 has changed. For example, latency of the wireless signal can be measured and used in combination with the resulting signal measured at the flexible inductor to determine how a configuration of the wearable device 200 has changed. Furthermore, latency of the wireless signal can be measured and used in combination with the resulting signal measured at the flexible inductor to predict where on the body of the person the wearable device 200 is located.

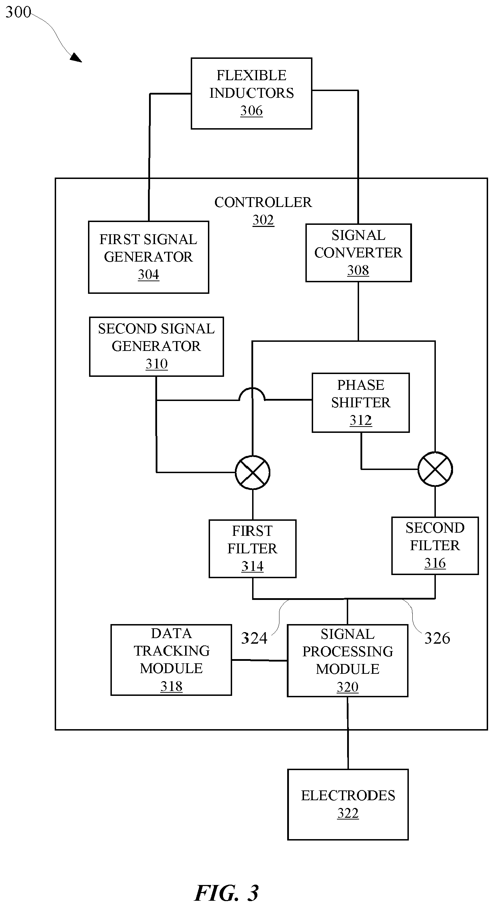

FIG. 3 illustrates a system diagram 300 of a controller 302 that can be included in any of the wearable devices discussed herein. The controller 302 can include a first signal generator 304 that generates (e.g., modulates) a signal that can be provided to one or more flexible inductors 306 of the wearable device. The signal can be a sinusoidal wave and the flexible inductors 306 can include one or more conductive coils that exhibit an alternating electromagnetic field when receiving the sinusoidal wave. The controller 302 can further include a signal converter 308, which can be embodied as software and/or hardware on the controller 302. The signal converter 308 can convert a resulting signal received from a flexible inductor 306 from an analog signal into a digital signal for analysis. The resulting signal can be an electrical signal that is induced in one of the flexible inductors 306. The resulting signal is induced in a flexible inductor 306 by another flexible inductor 306 when the other flexible inductor 306 receives the sinusoidal signal from the first signal generator 304. Because the flexible inductors 306 are flexible and can be stretched, compressed, and/or otherwise modified in their physical arrangement, the resulting signal can be different than the original sinusoidal signal. These differences can be analyzed by the controller 302 to determine how the physical arrangement of the wearable device has changed.

Once the resulting signal has been converted to a digital signal by the signal converter 308, the resulting signal can be further modified by splitting the resulting signal and feeding the split signals into two separate filters. However, it should be noted that the controller 302 can include a single filter or multiple other filters for analyzing the resulting signal. Prior to the resulting signal being received by the filters, a second signal generator 310 can generate a sinusoidal signal that can be added or subtracted to one of the resulting signals from the signal converter 308. In some embodiments, a frequency of the signal from the second signal generator 310 can be greater than or less than a frequency of the signal from the first signal generator 304. Furthermore, in some embodiments, the frequency of the signal from the second signal generator 310 can be proportional to the signal from the first signal generator 304.

A resulting signal from the signal converter 308, plus or minus the signal from the second signal generator 310 can be provided to a first filter 314 of the controller 302. The resulting signal from the signal converter 308, plus or minus a phase shifted signal from the second signal generator 310 can be provided to a second filter 316 of the controller 302. The resulting signal can be phase shifted by a phase shifter 312. The phase shifter 312 can be embodied as software and/or hardware in the controller 302. The phase shifter 312 can shift a phase of the resulting signal 90 degrees and/or any other angle suitable for isolating features of the resulting signal. Each of the first filter 314 and the second filter 316 can be low pass filters, high pass filters, band pass filters, and/or a combination thereof. For example, in some embodiments, both the first filter 314 and the second filter 316 are low pass filters. The first filter 314 and the second filter 316 can help to isolate features of the resulting signal or otherwise remove noise that might be present in the resulting signal.

Each signal from the first filter 314 and the second filter 316 can be received by a signal processing module 320 of the controller 302. A first signal 324 can correspond to a quadrature component of the resulting signal and a second signal 326 can correspond to an in-phase component of the resulting signal. The in-phase component can correspond to a real component and the quadrature component can correspond to an imaginary component. In some embodiments, the in-phase component and/or the quadrature component can be used by the signal processing module 320 to identify and track changes in the inductance of the flexible inductors 306. Inductance of the flexible inductors 306 can change differently with the movement of the body of the person 102 depending on the degree to which the flexible inductors 306 are being stretched during the movements. For example, changes in inductance that occur as a result of respiratory action when the flexible inductors 306 are at least partially surrounding the chest of a person 102 can be different than changes in inductance that occur when the flexible inductors 306 are wrapped around the waste of the person 102. Such differences can be identified by the signal processing module 320 to determine whether the wearable device is moving from its originally intended position on the body of the person 102. Furthermore, the changes in inductance can be used to identify movements of the person 102 for fitness tracking, vital signs of the person, and/or any other information helpful for monitoring a condition of the person 102.

The data inferred from the changes in the inductance and/or differences between the original signal and resulting signal can be tracked by a data tracking module 318 of the controller 302. For example, in some embodiments, an impedance of the flexible inductors 306 can be tracked over time to determine whether the flexible inductors 306 are degrading or otherwise losing the ability to transmit a signal. Furthermore, the data tracking module 318 can track a respiratory rate and/or heart rate of the person 102 over time. Data collected by the data tracking module 318 can be transmitted by the controller 302 to a peripheral device wirelessly in order to update the person 102 about the data being collected. Electrodes 322 can be employed by the wearable device for tracking conditions of the person 102 by transmitting signals between the body of the person 102 and the electrodes 322. Signals collected using the electrodes 322 can be analyzed by the signal processing module 320 and converted into data that can be tracked by the data tracking module 318. The data tracking module 318 can use the data collected based on operations by the electrodes 322 and the flexible inductors 306 to track conditions of the body of the person 102.

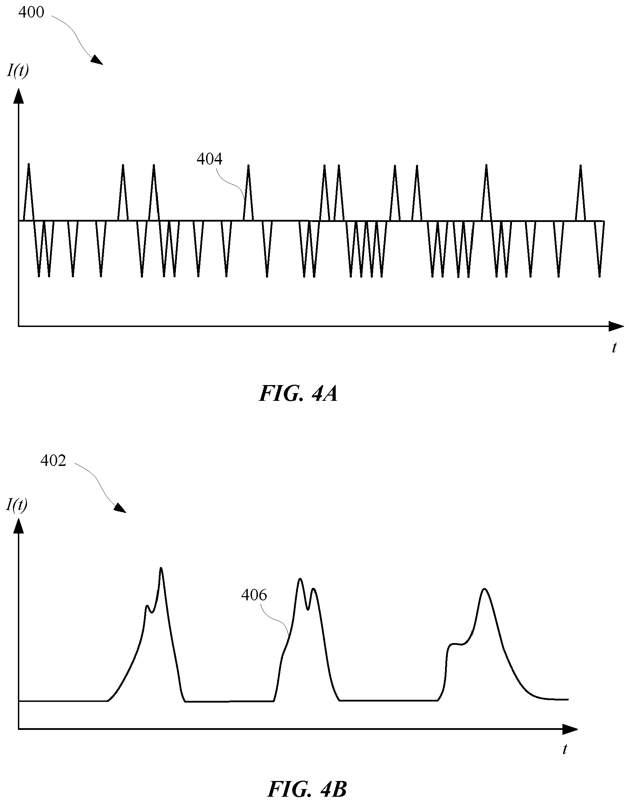

FIGS. 4A and 4B illustrate a plot 400 and a plot 402 of changes in inductance that can occur at one or more of the flexible inductors discussed herein. Specifically, plot 400 of FIG. 4A illustrates how inductance 404 (*I(t))* can nominally change over time when one or more of the flexible inductors is in a relaxed state. In other words, when one or more of the flexible inductors is attached to the body of a person and the flexible inductors are in a relaxed state, fluctuations in inductance 404 can be exhibited by the flexible inductors. These fluctuations in inductance 404 can be filtered in order to identify waveforms that are indicative of movements of the body of the person. For example, FIG. 4B illustrates a plot 402 of inductance waveforms 406 that occur as a result of a person breathing while the flexible inductors are attached to the body of the person and while the flexible inductors are in a relaxed state. The inductance waveforms 406 can be identified by filtering a resulting signal from one or more of the flexible inductors discussed herein. When a flexible inductor is receiving a sinusoidal signal in a relaxed state around a chest of a person, and the person expands and contracts their chest during breathing, the flexible inductor can also expand and contract. This expanding and contracting motion can affect the inductance and/or impedance of the flexible inductor, causing any resulting signal induced in another flexible inductor to be different than the original signal provided to the flexible inductor. These differences between the original signal and the resulting signal reflect the changes in inductance over time illustrated in FIG. 4B. Furthermore, as the resulting signal oscillates over time, a controller of the wearable device can analyze the resulting signal to determine a respiratory rate, and/or other metric that can be deduced from monitoring breathing over time. For example, estimates of oxygen intake can be generated by integrating the inductance waveforms 406 and isolating periods of the integrated inductance waveform corresponding to breaths of the person. Such estimates can be useful for identifying whether a person's oxygen intake is increasing or decreasing over time. If an estimate of oxygen intake decreased to below a threshold set by the controller of the wearable device, the wearable device can transmit an emergency signal. The emergency signal can be an audio signal or wireless signal to a peripheral device for alerting a medical provider about the person's condition.

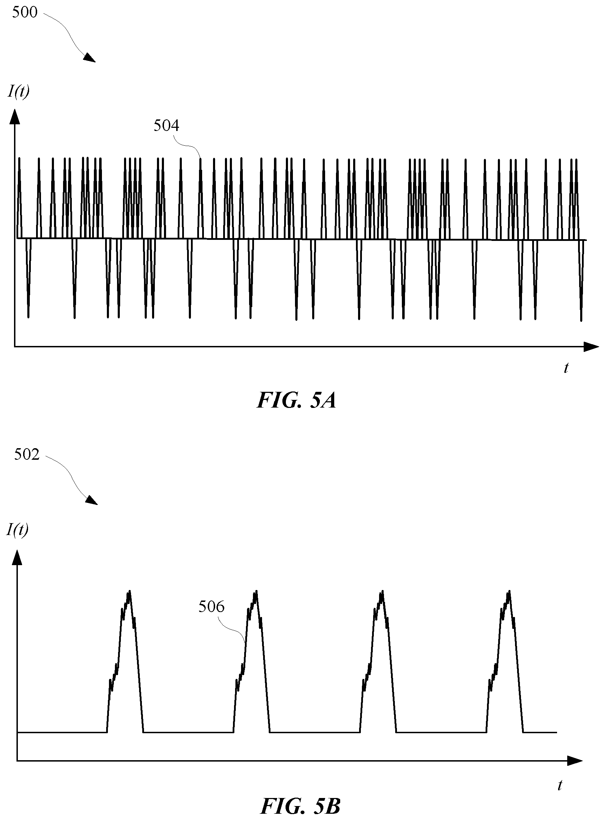

FIGS. 5A and 5B illustrate plots 500 and 502 that can represent changes in inductance (I(t)) that can occur when one or more flexible inductors of a wearable device are in a stretched state. Specifically, FIG. 5A illustrates a plot 500 of changes in inductance 504 that can occur at a flexible inductor that is stretched about a body of a person. Compared to plot 400, a flexible inductor that is in a stretched state can exhibit more frequent nominal changes in inductance. Therefore, a controller of the wearable device can identify a frequency of nominal changes in inductance 504 to determine whether one or more flexible inductors of the wearable device is in a stretched state. When the one or more flexible inductors is in a stretched state, a specific algorithm can be used for identifying bodily movements of a person. This algorithm can be different than or the same as an algorithm that can be used to identify bodily movements when the one or more flexible inductors is in a relaxed state. For example, FIG. 5B can correspond to respiratory movements of a person that is wearing the wearable device on and/or around their chest while one or more flexible inductors of the wearable device are stretched at least partially on and/or around their chest. While stretched, a flexible inductor can receive a signal that causes the flexible inductor to induce a resulting signal in another flexible signal. If the person wearing the wearable device is breathing, the original signal and the resulting signal will have different characteristics, which can change according to the breathing of the person. Such changes can be an inductance and/or an impedance of the flexible inductors, as provided in FIG. 5B, which illustrates how the inductance 506 can rise and fall with the respiratory action of a person. Over time, the inductance 506 can be tracked to identify a respiratory rate of a person wearing the wearable device. Respiratory action that causes fluctuations in inductance 506 when the flexible inductors are stretched can result in sharper changes in inductance 506, as provided in FIG. 5B. These sharper changes can be identified by the controller and used to track respiratory action while flexible inductors are in a stretched state.

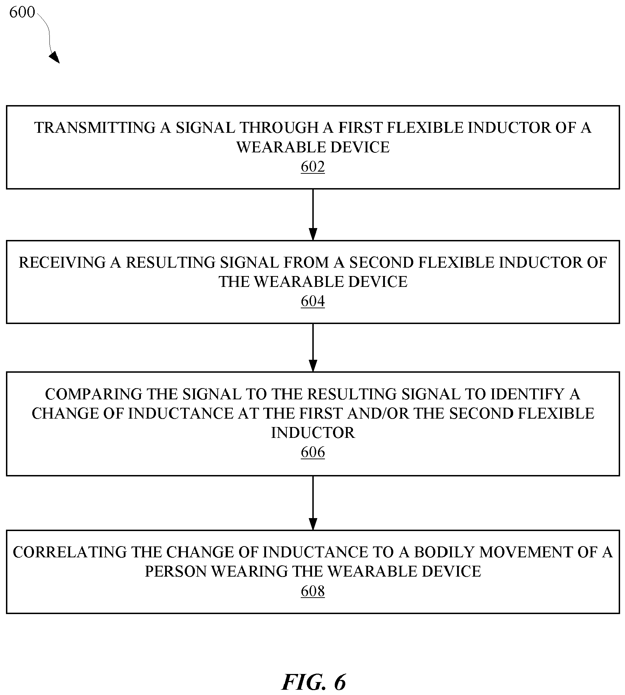

FIG. 6 illustrates a method 600 for determining how a person is moving based on changes of inductance that can occur at a flexible inductor of a wearable device. The method 600 can be performed by one or more computing devices, controllers, processors, and/or apparatuses suitable for controlling the wearable device. The method 600 can include a block 602 of transmitting a signal through a first flexible inductor of a wearable device. The wearable device can be a health monitoring device that tracks different metrics about the body of a person wearing the wearable device. The wearable device can include a first flexible inductor and a second flexible inductor that can extend over the body of the person. At block 604, a resulting signal can be received from the second flexible inductor of the wearable device. The resulting signal can be induced in the second flexible inductor by the first flexible inductor. At block 606, the signal and the resulting signal are compared to identify a change of inductance that has occurred at the first flexible inductor and/or the second flexible inductor. The change of inductance can occur as a result of a physical force moving the wearable device. At block 608, the change of inductance can be correlated to a bodily movement of the person wearing the wearable device. For example, the bodily movement can be a step taken by the person, a respiratory motion of the person, and/or any other bodily movement that can be tracked by the motion of an electronic device.

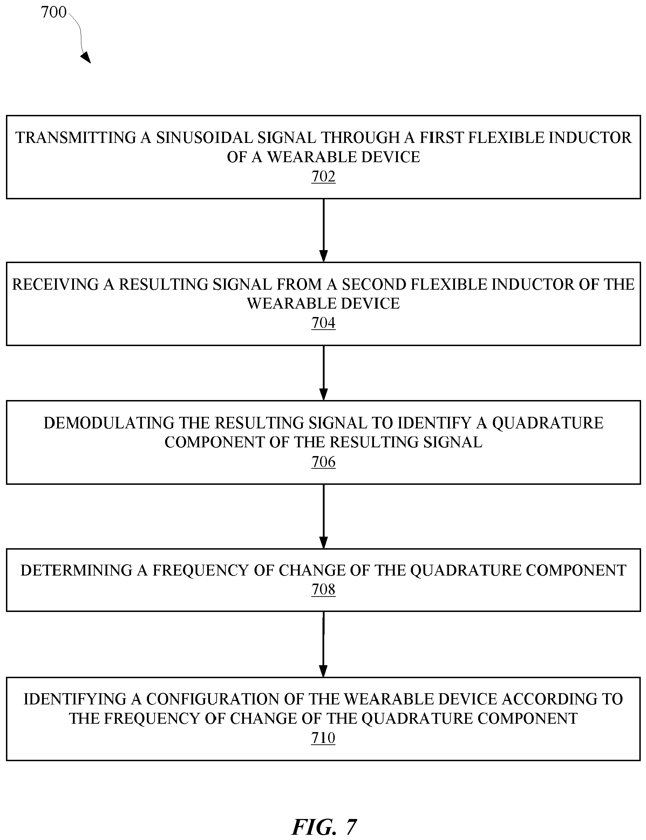

FIG. 7 illustrates a method 700 for identifying a configuration of a wearable device using one or more flexible inductors of the wearable device. The method 700 can be performed by one or more computing devices, controllers, processors, and/or apparatuses suitable for controlling the wearable device. The method 700 can include a block 702 of transmitting a sinusoidal signal through a first flexible inductor of the wearable device. At block 704, a resulting signal from a second flexible inductor of the wearable device can be received. The resulting signal can be induced in the second flexible inductor by the first flexible inductor when the first flexible inductor is receiving the sinusoidal signal. At block 706, the resulting signal is demodulated to determine a quadrature component of the resulting signal. The quadrature component can refer to real and imaginary values for the resulting signal. The imaginary values can be a quadrature phase of the resulting signal and be used to determine inductance of the first and/or second flexible inductor. At block 708, a frequency of change of the quadrature component is determined. The frequency of change can be determined by tracking the quadrature component over a period of time and analyzing how often the quadrature component changes over time. At block 710, a configuration of the wearable device is identified according to the frequency of change of the quadrature component. For example, a frequency of change of the quadrature component can be higher when the wearable device is worn around the chest rather than worn around the arm or leg of a person. Furthermore, the wearable device can include a memory storing a lookup table that correlates different frequencies with different configurations of the wearable device. In response to identifying a configuration of the wearable device, an operation of the wearable device can change according to which configuration has been identified. For example, the wearable device can track respiratory data when it is determined that the wearable device is being worn around the chest, and track arm movement repetitions when the wearable device is being worn around the arm.

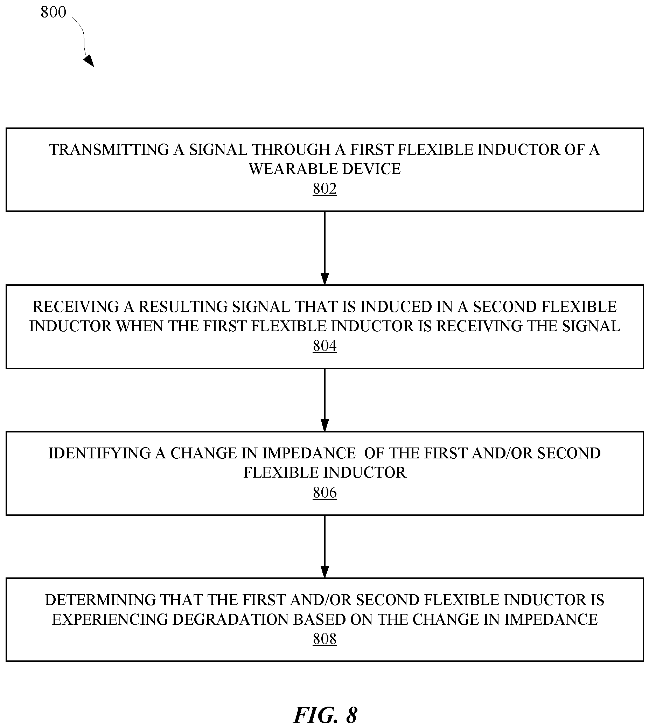

FIG. 8 illustrates a method 800 for determining whether one or more flexible inductors of a wearable device are experiencing degradation. The method 800 can be performed by one or more computing devices, controllers, processors, and/or apparatuses suitable for controlling the wearable device. The method 800 can include a block 802 of transmitting a signal through a first flexible inductor of a wearable device. The first flexible inductor can be a coil of wire that extends through and/or over a layer of the wearable device. At block 804, a resulting signal is received from a second flexible inductor when the first flexible inductor is receiving the signal. The second flexible inductor can extend through the first flexible inductor, or otherwise be proximate enough to the first flexible inductor that the resulting signal is induced in the second flexible inductor. At block 806, a change in impedance of the first flexible inductor and/or the second flexible inductor is identified. The change in impedance can be identified by measuring an impedance of the first flexible inductor and/or the second flexible inductor over a period of time. At block 808, it can be determined that the first flexible inductor and/or the second flexible inductor are experiencing degradation based on the change in impedance. When the determination is made that the first flexible inductor and/or the second flexible inductor is experiencing degradation, a notification signal can be transmitted from the wearable device to warn a user of the degradation. For example, in some embodiments, the signal may be transmitted (e.g., wirelessly) to a peripheral device (e.g., smart watch, smart phone, standalone smart-speaker, etc.) that is tasked with tracking the degradation of the wearable device over time. The peripheral device may then render output (e.g., audio, visual, haptic, etc.) that notifies the user of the degradation, and in some instances, that a contact is loosely coupled and/or has degraded. In other embodiments in which the wearable device is a wearable computing device such as a smart watch, smart band, smart belt, etc., the wearable device itself may raise an alarm, in addition to or instead of transmitting a signal to a peripheral device.

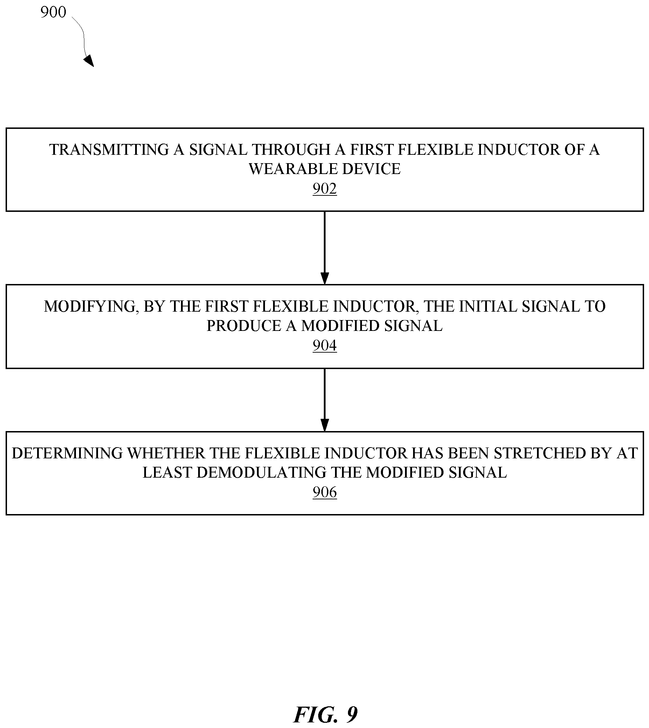

FIG. 9 illustrates another example method for determining whether one or more flexible inductors of a wearable device are experiencing degradation. At block 902, a controller may transmit a signal through a first flexible inductor of a wearable device. At block 904, the flexible inductor may, e.g., in response to being stretched while receiving the initial signal, modify the initial signal to produce a modified signal. At block 906, the controller may determine whether the flexible inductor has been stretched by at least demodulating the modified signal.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope

## Claims

1. A wearable apparatus (104, 200), comprising:
a flexible inductor (108, 116, 216, 306) configured to modify an initial signal to produce a modified signal (404, 504) when the flexible inductor is stretched while receiving the initial signal;
a controller (118, 220, 302) configured to transmit the initial signal to the flexible inductor and determine whether the flexible inductor has been stretched by at least demodulating the modified signal; and
a flexible body (212) configured to at least partially envelope the flexible inductor and the controller.

2. The wearable apparatus of claim 1, further comprising:
a memory (302) that is connected to the controller, the memory configured to receive respiratory rate data from the controller when a length of the flexible inductor oscillates over time.

3. The wearable apparatus of claim 1, further comprising:
a conductive contact (204, 206) located at an exterior surface of the flexible body, the conductive contact configured to receive a monitoring signal from the controller, wherein the controller is further configured to determine when the conductive contact has shifted from an original position on a person by monitoring a change of impedance of the flexible inductor.

4. The wearable apparatus of claim 1, wherein the controller is further configured to determine whether a material of the flexible inductor is degrading based on impedance measurements of the flexible inductor at different times.

5. The wearable apparatus of claim 4, further comprising:
a wireless transmitter configured to output a warning signal to a computing device when the impedance measurements exceed a threshold impedance value for a period of time.

6. The wearable apparatus of claim 1, further comprising:
a wireless transmitter configured to output a wireless signal to a computing device when the controller determines that the flexible inductor has been stretched past a predetermined length, wherein the wireless signal indicates an arrangement of the flexible inductor to the computing device.

7. The wearable apparatus of claim 1, wherein the controller is further configured to determine, at least partially based on the demodulating of the modified signal, a length by which the wearable apparatus is being stretched.

8. The wearable apparatus of claim 7, further comprising:
a sensor (204, 206), wherein the controller is further configured to modify a data collection operation of the sensor when the length satisfies a predetermined threshold.

9. The wearable apparatus of claim 8, wherein determining the length includes determining a change of inductance of the flexible inductor when the flexible inductor is stretched.

10. The wearable apparatus of claim 8, further comprising:
a memory, wherein the controller is further configured to modify a rate value stored in the memory when the length exceeds the predetermined threshold, wherein the rate value indicates an average number of times the wearable apparatus is stretched within a period of time.

11. The wearable apparatus of claim 1, further comprising:
at least two electrodes (204, 206) connected to the controller and located at opposite ends of the flexible body, wherein the at least two electrodes become less proximate when the flexible inductor is stretched.

12. The wearable device of claim 11, wherein the controller is further configured to simultaneously monitor a vital sign of a person using the electrodes and track the stretching of the flexible inductor.

13. The wearable device of claim 11, wherein the controller is further configured to determine a frequency of the change of inductance of the flexible inductor and determine whether the at least two electrodes have separated beyond a threshold distance based on the frequency of the change of inductance.

14. The wearable device of claim 13, wherein the controller is further configured to change an operation of the at least two electrodes when the at least two electrodes have separated beyond the threshold distance.

15. The wearable device of claim 1, further comprising another flexible inductor that carries the modified signal when the flexible inductor is receiving the initial signal.

16. A method (900) of using a wearable device (104, 200) that includes a flexible body (212) that at least partially envelopes a flexible inductor (108, 116, 216, 306) and a controller (118, 220, 302), the method comprising:
transmitting (902), by the controller, an initial signal to the flexible inductor;
modifying (904), by the flexible inductor, the initial signal to produce a modified signal (404, 504) when the flexible inductor is stretched while receiving the initial signal; and
determining(906), by the controller, whether the flexible inductor has been stretched by at least demodulating the modified signal.
